# EUROPEAN PATENT SPECIFICATION

(11) **EP 3 068 345 B1**
(45) Date of publication and mention of the grant of the patent: **26.08.2020**
(21) Application number: 14816480.9
(22) Date of filing: 14.11.2014
(51) Int. Cl.: A61F 2/24

(54) **INFORMATION MARKERS FOR HEART PROSTHESES**
INFORMATIONSMARKER FÜR HERZPROTHESEN
MARQUEURS D'INFORMATION POUR PROTHÈSES CARDIAQUES

(30) Priority: 15.11.2013 US 201361904565 P
(43) Date of publication of application: 21.09.2016
(73) Proprietor: Guy's And St. Thomas' NHS Foundation Trust, London SE1 7EH (GB); Medtronic, Inc., Minneapolis, MN 55432 (US)
(72) Inventor: BAPAT, Vinayak, London SE1 7EH (GB); RYAN, Timothy, Minneapolis, MN 55432 (US)
(74) Representative: Zimmermann & Partner Patentanwälte mbB
(86) International application number: PCT/US2014/065692
(87) International publication number: WO 2015/073815

(56) References cited:
- EP-A1- 2 617 390
- WO-A1-96/22058
- WO-A1-98/29146
- WO-A1-2009/094501
- WO-A1-2010/031060
- WO-A1-2015/196152
- WO-A2-2015/013536
- CN-A- 102 760 240
- US-A1- 2007 018 810

## Description

### BACKGROUND

Diseased or otherwise deficient heart valves can be repaired or replaced using a variety of different types of heart valve surgeries. Typical heart valve surgeries involve an open-heart surgical procedure that is conducted under general anesthesia, during which the heart is stopped while blood flow is controlled by a heart-lung bypass machine. This type of valve surgery is highly invasive and exposes the patient to a number of potentially serious risks, such as infection, stroke, renal failure, and adverse effects associated with use of the heart-lung machine, for example.

Recently, there has been increasing interest in minimally invasive and percutaneous replacement of original heart prostheses, e.g., prosthetic heart valves, annuloplasty prostheses, etc. Such surgical techniques involve making a very small opening in the skin of the patient into which a valve assembly is inserted in the body and delivered to the heart via a delivery device similar to a catheter. This technique is often preferable to more invasive forms of surgery, such as the open-heart surgical procedure described above. In the context of pulmonary valve replacement, U.S. Patent Application Publication Nos. 2003/0199971 A1 and 2003/0199963 A1, both filed by Tower, et al., describe a valved segment of bovine jugular vein, mounted within an expandable stent, for use as a replacement pulmonary valve. The replacement valve is mounted on a balloon catheter and delivered percutaneously via the vascular system to the location of the failed pulmonary valve and expanded by the balloon to compress the valve leaflets against the right ventricular outflow tract, anchoring and sealing the replacement valve. As described in the articles "Percutaneous Insertion of the Pulmonary Valve," Bonhoeffer, et al., Journal of the American College of Cardiology 2002; 39: 1664-1669 and "Transcatheter Replacement of a Bovine Valve in Pulmonary Position," Bonhoeffer, et al., Circulation 2000; 102: 813-816, the replacement pulmonary valve may be implanted to replace native pulmonary valves or prosthetic pulmonary valves located in valved conduits.

Various types and configurations of prosthetic heart valves are used in valve procedures to replace diseased natural human heart valves. The actual shape and configuration of any particular prosthetic heart valve is dependent to some extent upon the valve being replaced (i.e., mitral valve, tricuspid valve, aortic valve, or pulmonary valve). In general, the prosthetic heart valve designs attempt to replicate the function of the valve being replaced and thus will include valve leaflet-like structures used with either bioprostheses or mechanical heart valve prostheses.

Percutaneously-delivered replacement valves may include a valved vein segment that is mounted in some manner within an expandable stent to make a stented valve. To prepare such a valve for percutaneous implantation, the stented valve can be initially provided in an expanded or uncrimped condition, then crimped or compressed around the balloon portion of a catheter until it is as close to the diameter of the catheter as possible.

Other percutaneously-delivered prosthetic heart valves have been suggested having a generally similar configuration, such as by Bonhoeffer, P. et al., "Transcatheter Implantation of a Bovine Valve in Pulmonary Position," Circulation, 2002; 102:813-816, and by Cribier, A. et al. "Percutaneous Transcatheter Implantation of an Aortic Valve Prosthesis for Calcific Aortic Stenosis," Circulation, 2002; 106:3006-3008. These techniques rely at least partially upon a frictional type of engagement between the expanded support structure and the native tissue to maintain a position of the delivered prosthesis, although the stents can also become at least partially embedded in the surrounding tissue in response to the radial force provided by the stent and balloons that are sometimes used to expand the stent. Thus, with these transcatheter techniques, conventional sewing of the prosthetic heart valve to the patient's native tissue is not necessary.

Similarly, in an article by Bonhoeffer, P. et al. titled "Percutaneous Insertion of the Pulmonary Valve," J Am Coll Cardiol, 2002; 39:1664-1669, percutaneous delivery of a biological valve is described. The valve is sutured to an expandable stent within a previously implanted valved or non-valved conduit, or a previously implanted valve. Again, radial expansion of the secondary valve stent is used for placing and maintaining the replacement valve.

When replacing an implanted heart prosthesis using these percutaneous techniques, the physician or clinician needs to know certain characteristics of the original implanted prosthesis so that a replacement valve can be selected that is qualified for use with the particular original prosthesis. For example, information such as the manufacturer, type, model, feature, size, date, or other characteristic of the original implanted prosthesis can guide the physician or clinician in selecting the appropriate replacement valve.

WO 96/22058 A1 describes a living tissue implant with electrical transponder.

WO 98/29146 A1 describes indicia for a prosthetic device.

WO 2009/094501 A1 describes markers for prosthetic heart valves.

WO 2010/031060 A1 describes a prosthetic heart valve having identifiers for aiding in radiographic positioning.

US 2007/018810 A1 describes radio frequency identification and tagging for implantable medical devices and medical device systems.

### SUMMARY

The present invention is defined in appended claim 1.

### BRIEF DESCRIPTION OF THE DRAWINGS

Throughout the specification, reference is made to the appended drawings, where like reference numerals designate like elements, and wherein:
FIG. 1 is a schematic side view of an original prosthetic heart valve.
FIG. 2 is a schematic side view of one embodiment of a replacement prosthetic heart valve.
FIG. 3 is a schematic plan view of the replacement prosthetic heart valve of FIG. 2.
FIG. 4 is a schematic side view of the replacement prosthetic heart valve of FIG. 2 positioned relative to the original prosthetic heart valve of FIG. 1.
FIG. 5 is a schematic plan view of the valve and valve frame of FIG. 4.
FIG. 6 is a schematic perspective view of an annuloplasty prosthesis.
FIG. 7 is a schematic side view of the replacement prosthetic heart valve of FIG. 2 positioned relative to the annuloplasty prosthesis of FIG. 6.

### DETAILED DESCRIPTION

In general, the present disclosure provides various embodiments of heart prostheses that include at least one information marker. This information marker can, in some embodiments, be detected using any suitable clinical imaging techniques such as fluoroscopy, magnetic resonance imaging (MRI), echocardiogram, etc. The information marker can provide a physician or clinician information regarding an implanted original heart prosthesis, e.g., one or more of a manufacturer, type, model, feature, size, and date associated with the heart prosthesis. This information can aid the clinician in determining which replacement prosthetic heart valve can be implanted in a patient such that it is compatible with the original heart prosthesis or has been qualified for re-intervention with the particular original heart prosthesis.

These information markers can also be valuable in emergency situations to aid a physician or other health care provider determine whether a patient has an implanted heart prosthesis, and if so, the manufacturer, model, etc. of the prosthesis, and whether any special precautions may be needed in treating the patient in the emergency situation in light of the implanted prosthesis.

Markers may convey information in several ways. For instance, a shape included as part of the marker may be defined, in part, by "cutaway" portions of radiopaque material. As a specific example, a manufacturer's logo may be cut out of an otherwise continuous surface of radiopaque material so that an image of this cutaway portion can be used to determine the manufacturer of the product. Other cutaway shapes may indicate the model of the heart valve, and so on.

In some embodiments, when imaging technology is used to view such a marker, the cutaway portions appear as a "negative" image of the information to be conveyed. That is, it is the absence of portions of the radiopaque material (i.e., the "cutaway" portions), rather than the presence of such material, that serves to convey information.

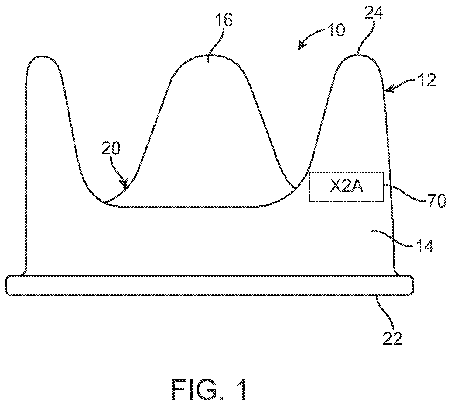

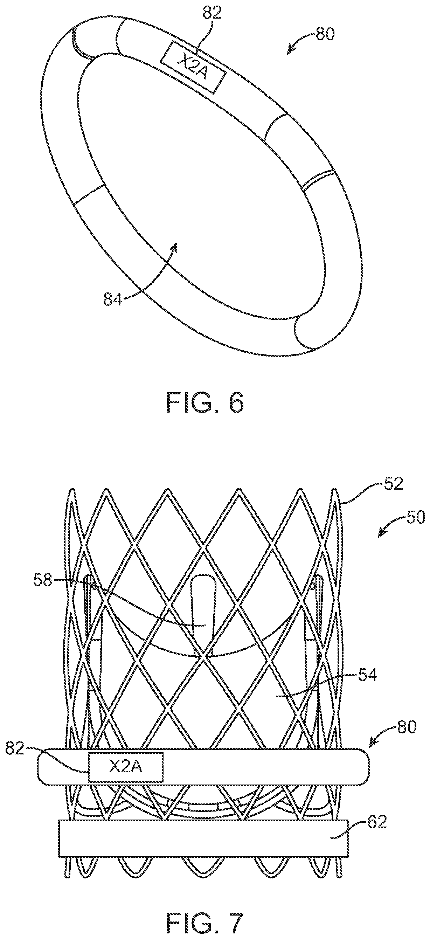

In general, the various disclosed embodiments of information markers can be included with any suitable heart prosthesis. For example, in some embodiments, the original heart prosthesis can include an original prosthetic heart valve, e.g., prosthetic heart valve 10 of FIG. 1. In other embodiments, the original heart prosthesis can include an annuloplasty prosthesis, e.g., annuloplasty prosthesis 80 of FIG. 6.

The features of the disclosure can be used for aortic valve, mitral valve, pulmonic valve, venous, gastric, and/or tricuspid valve replacement. In some embodiments, the prosthetic heart valves of the disclosure are highly amenable to transvascular delivery (either with or without cardiopulmonary bypass and either with or without rapid pacing). The methodology associated with the present disclosure can be repeated multiple times, such that several heart prostheses of the present disclosure can be mounted on top of or within one another, if necessary or desired. Where dimensions are noted in inch, the conversion is 1 inch = 2.54 cm.

FIG. 1 is a schematic side view of a prosthetic heart valve 10. The valve 10 can be an original prosthetic heart valve if it has been implanted in a patient prior to replacement with a replacement prosthetic heart valve as described herein. The valve 10 is a typical configuration of a valve that can be implanted within the heart of a patient, such as by suturing or otherwise securing the valve 10 into the area of a native heart valve of a patient. The native heart valves referred to herein can be any of the human heart valves (i.e., mitral valve, tricuspid valve, aortic valve, or pulmonary valve), where the type and orientation of an implanted (e.g., surgically implanted) prosthetic heart valve 10 will correspond with the particular form, shape, and function of the native heart valve in which it is implanted. Although valve 10 would typically include multiple leaflets attached within its interior area, such leaflets are not shown in this figure for illustration clarity purposes. Prosthetic heart valve 10 can be any suitable heart valve, e.g., a surgically implanted prosthetic heart valve, a transcatheter prosthetic heart valve, etc.

Valve 10 generally includes a valve structure 12 including a stent structure 14 from which multiple stent posts or commissure posts 16 extend. All or a portion of the valve structure 12, including the stent structure 14 and stent posts 16, can be covered by a flexible covering, which may be a tissue, polymer, fabric, cloth material, or the like to which leaflets (not shown) of the heart valve 10 are attached, such as by sewing. The stent structure 14 may alternatively be a wire form. Further, as is known in the art, the internal structure of each of the stent posts 16 can be formed of a stiff but somewhat resiliently bendable material. This construction allows the stent posts 16 to be moved from the orientation shown in FIG. 1 to a deflected orientation by the application of an external force. Once this external force is removed or reduced, the stent posts 16 can then move back toward the orientation shown in FIG. 1. Alternatively, the stent posts can be angled at least slightly toward or away from a central axis of the valve 10.

The valve structure 12 is generally tubular in shape, defining an opening or internal area 20 (referenced generally) that extends from an inflow end 22 to an outflow end 24. The opening 20 is essentially surrounded by the valve structure 12, and the leaflets attached within the valve structure 12 selectively allow for fluid flow into and out of the lumen of the natural heart valve in which it is implanted. That is, the opening 20 is alternatively open and closed to the lumen of the natural heart valve in which it is inserted via movement of leaflets.

In some patients, the prosthetic heart valve 10 will be implanted using typical surgical techniques, whereby the stent ring 14 is sewn or attached to the annulus or valvular rim of the native heart valve. Alternatively, the prosthetic valve can be placed in the patient using minimally invasive techniques for holding the valve in place, such as U-clips, for example, or a wide variety of other techniques and features used for minimally invasive and/or percutaneous implantation of the initial prosthetic heart valve.

The prosthetic heart valves (e.g., heart valve 10 and replacement prosthetic heart valve 50 that will be discussed herein) used in accordance with the devices and techniques of the disclosure can include a wide variety of different configurations, such as a prosthetic heart valve that has tissue leaflets, or a synthetic heart valve that has polymeric leaflets. In this way, the heart valves can be specifically configured for replacing any heart valve.

In the illustrated example, the valve 10 includes at least one information marker 70. The at least one information marker 70 can be positioned in any suitable location on or in the valve 10, e.g., on the stent structure 14 or a sealing skirt of the valve. According to the invention, the information marker is positioned on a sealing skirt of the prosthetic heart valve. Further, the at least one information marker 70 can include any suitable information. For example, in some embodiments, the at least one information marker 70 indicates one or more of a manufacturer, type, model, feature, size, and date associated with the prosthetic heart valve 10 using one or more articles or indicia of any suitable size such that the indicia can be detected by a physician using suitable visualization techniques. The at least one information marker 70 can be formed using any suitable technique and include any suitable materials as is further described herein. In some embodiments, the at least one information marker 70 can be radiopaque.

Although the valve 10 of FIG. 1 includes one information marker 70, any suitable number of information markers can be included. In some embodiments, two or more information markers 70 are included, where each of the two or more information markers are the same, e.g., convey the same information. In other embodiments, each of the two or more information markers 70 can include different information. For example, one information marker can include information regarding the manufacturer of the valve 10, and another information marker can include information regarding the date the valve was manufactured or surgically implanted.

As shown in FIG. 1, the at least one information marker 70 includes multiple individual articles or indicia shown as "X," "2," and "A." More or fewer such articles may be included in marker 70. In the illustrated embodiment, these articles are shown arranged in a character string. In other examples, marker 70 can have the individual articles arranged in another manner, such as in a two-dimensional array of characters or in some other two-dimensional pattern. In some examples, the marker 70 can include a three-dimensional arrangement of articles such that not all of the articles are aligned on a same plane as all other markers. This may be useful in allowing the marker to be viewed from multiple angles, as when the valve 10 is in various positions or orientations relative to an imaging device. In some examples, the at least one information marker 70 can include multiple instances of a set of articles, with each set of articles being arranged in a different plane to make information viewable from multiple directions.

As discussed herein, the position occupied by a particular article may assign significance to that article. For instance, a first one or more articles in a sequential string of articles (e.g., article "X") may be designated to denote a model of the prosthetic heart valve 10. A second one or more articles in a sequential string of articles may denote a feature set of the valve 10, and so on. When a two- or three-dimensional array or other pattern is used to form the at least one information marker 70, the position of an article within the array or other pattern can likewise assign a particular significance to the article. In this manner, not only the article itself, but also the position of the article, may be used to convey information associated with the valve 10.

In the example illustrated in FIG. 1, each of the articles included in the at least one information marker 70 is an alphanumeric character. In some examples, the marker 70 can alternatively or additionally include any other types of symbols or geometric shapes. Such symbols may be predefined (e.g., #, %, @, etc.) or may be entirely arbitrary (e.g., symbols defined by a manufacturer such as a logo of a manufacturer.) In the claimed invention, the at least one marker 70 comprises a QR code.

In some examples, each of the articles in the set of articles used to form the marker 70 can have similar characteristics, such as being made via a common manufacturing process, being formed of a same material, having roughly a same size (e.g., length, width, shape, and/or material thickness), having similar feature(s) used to affix or retain a position and/or orientation of the article, and so on. Having common characteristics (e.g., size) may allow a selected combination of the articles to be more readily incorporated within a same marker.

The symbols in the marker 70 of FIG. 1 may be said to provide a "positive" outline of the information to be conveyed. As described herein, this means that the material used for the marker 70 forms the actual cutout characters. As a specific illustration, the characters "X," "2," and "A," of this example are cut out of, or otherwise formed from, a radiopaque material. The remainder of marker 70 (that is, the object that carries the radiopaque articles) may be formed of a non-radiopaque material such as a polymer. This positive image of the information is in contrast to a negative image, where portions of a radiopaque material are cutaway to provide information. As a specific example, the letters "A," "B," and "C" may be cut out of a sheet of radiopaque material so that when imaging technology is used to view marker 70 this cutaway image is visible. This is akin to shining a light through a window into a dark room such that the outline of the window may be visible on an adjacent wall. While either type of image is contemplated herein in various embodiments, the use of a positive image of the type shown in FIG. 1 may provide a marker 70 that is more readily discernible, particularly when the marker is relatively small.

As previously discussed herein, a marker that includes one or more articles selected from a set of such articles can convey information in a number of ways. First, each article selected for inclusion in the marker may convey information by virtue of that article's unique shape, size, and/or other physical characteristics. For example, an article formed like the letter "M" has a unique shape that may be assigned a particular meaning (e.g., "this device is MRI conditionally safe"). Similarly, an article formed in the shape of a manufacturing logo may be used to convey the manufacturer of the device. A different article assigned some arbitrary shape may be associated with a model of a heart valve. In this example, the ordering or other arrangement of the articles within the marker may not be very important, since each unique article included in the marker is used to convey the necessary information.

In some examples, the spatial relationship of articles included in the at least one information marker can be important. For instance, an information marker can include a string of three articles "MM1" arranged in a string from back-to-front on a heart valve. The first article "M" in this string may indicate the make of the valve. The next article "M" in the string can indicate a model of the valve, and the third article "1" in the string may identify a feature set of the valve. Thus, even though two articles in the marker are the same (i.e., "M"), the articles take on a different significance based on the spatial arrangement in the marker. In yet another example, the first two characters "MM" may be assigned a certain meaning indicative of the feature set of the valve. Thus, in this example, both the spatial arrangement and the articles selected for inclusion within the marker provide information associated with the heart valve.

In other embodiments, the spatial arrangement may have a two-dimensional or even a three-dimensional aspect that may also convey information in some instances. For example, a multi-shot molding process may be used to add a three-dimensional quality to an information marker. A shape of the three-dimensional marker and/or locations of the articles within the three dimensions may be used to convey information.

A three-dimensional marker may be useful, for example, when the orientation of a heart valve is unknown such that the marker is readable from various directions. In one instance, a three-dimensional marker may utilize multiple radiopaque articles to convey the same information in multiple planes. For instance, two articles "M," both of which convey the manufacturer of the device, may be arranged to lie in two substantially-perpendicular planes within the same three-dimensional marker. This may make it easier for an imaging device to read at least one of the articles when the orientation of the heart valve within the patient is unknown.

In some embodiments, the at least one information marker 70 can be made of a material that allows it to be viewed from the opposite surface of the stent post from the surface on which the marker is placed (i.e., "through" the stent post) when using certain imaging techniques. Due to the directional nature of the markers, these indicia would therefore be displayed backwards or as a mirror image of the original marker when viewed from the opposite side of the commissure post. However, in some embodiments, the marker 70 may not be visible to the unassisted eye in this "backward" orientation, but that it will only be visible in this orientation when using specific visualization equipment. In other embodiments, the markers) can extend through the entire thickness of the stent or are provided in some other way so that they are visible from both sides, even without visualization equipment. In other words, any suitable orientation can be utilized with marker 70 such that it can either be visible or not visible when in a backward orientation.

In some embodiments, the at least one information marker 70 provided on or in the heart valve can be made of a radiopaque material and/or have echogenic or other properties so that it is visible from outside the patient's body when using an appropriate imaging technique. The marker 70 can be made of platinum iridium, tungsten, barium sulfate, other radiopaque materials, and the like. In some embodiments, marker 70 can also be constructed from materials impregnated with radiopaque or echogenic materials, including fabric sutures or elastomers such as silicone. In this way, the marker 70 can be used to provide selected information associated with the heart valve.

The at least one information marker 70 can be provided on or in any surface of a heart valve using any suitable technique. In some embodiments, the marker 70 can be directly deposited onto a surface of the valve. In other embodiments, the marker 70 can first be formed as described herein and then attached to a surface of the valve using any suitable technique. Also, in some embodiments, these preformed markers can be inserted into openings in the stent frame.

After some period of time, it may become desirable to place or implant a replacement prosthetic heart valve relative to a previously implanted prosthetic heart valve to functionally replace the older heart valve. This may occur in cases where it is determined that a previously implanted or repaired prosthetic heart valve is functionally deficient due to one or more of a variety of factors, such as stenosis, valve failure, structural thrombosis, inflammation, valve insufficiency, and/or other medical conditions. Regardless of the cause of the deficiency, rather than removing the previously implanted prosthetic heart valve and implanting a second, similarly configured prosthetic heart valve via relatively complicated and invasive open heart surgical techniques, some embodiments of the present disclosure leave the deficient previously implanted or repaired prosthetic heart valve in place (e.g., original prosthetic heart valve 10), and deploy a replacement heart valve so that it functionally replaces the previously implanted prosthetic heart valve. Prior to implanting the replacement valve, the leaflets of the previously implanted and deficient prosthetic heart valve can either be removed using a variety of techniques such as cutters, lasers, and the like, or the leaflets may instead be left in place within the deficient valve, where they will likely be pushed toward the walls of the vessel upon implantation of the replacement valve or pushed out prior to replacement, e.g., with a balloon to increase the size of the orifice.

A number of factors can be considered, alone or in combination, to verify that the valve is properly placed in an implantation site, where some exemplary factors are as follows: (1) lack of paravalvular leakage around the replacement valve, which can be advantageously examined while blood is flowing through the valve since these delivery systems allow for flow through and around the valve; (2) optimal rotational orientation of the replacement valve relative to the coronary arteries; (3) the presence of coronary flow with the replacement valve in place; (4) correct longitudinal alignment of the replacement valve annulus with respect to the native patient anatomy; (5) verification that the position of the sinus region of the replacement valve does not interfere with native coronary flow; (6) verification that the sealing skirt is aligned with anatomical features to minimize paravalvular leakage; (7) verification that the replacement valve does not induce arrhythmias prior to final release; (8) verification that the replacement valve does not interfere with function of an adjacent valve, such as the mitral valve; and (9) verification of normal cardiac rhythm.

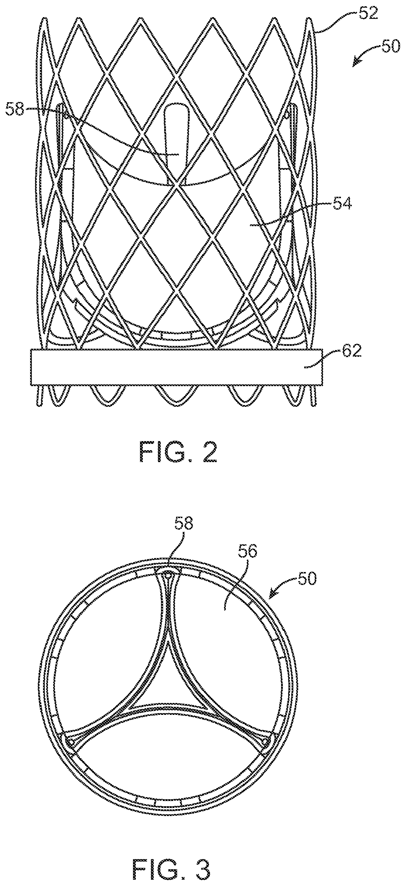

FIGS. 2-3 illustrate one exemplary embodiment of a replacement prosthetic heart valve 50. The valve 50 includes a stent structure 52 and a valve structure 54 positioned within and attached to the stent 52. The valve 50 further includes a sealing skirt 62 adjacent to one end that extends generally around the outer periphery of the stent 52. In general, the stents described herein include a support structure including a number of strut or wire portions arranged relative to each other to provide a desired compressibility and strength to the heart valve. Other details of various configurations of the stents of the disclosure are also described herein; however, in general terms, stents of the disclosure are generally tubular support structures, and a valve structure will be secured with this support structure to make a stented valve.

Some embodiments of the support structures of the stents described herein can be a series of wires or wire segments arranged so that they are capable of transitioning from a collapsed state to an expanded state. The stents may further include a number of individual wires formed of a metal or other material that include the support structure. These wires are arranged in such a way that allows for folding or compressing to a contracted state in which the internal stent diameter is greatly reduced from when it is in an expanded state. In its collapsed state, such a support structure with attached valves can be mounted over a delivery device, such as a balloon catheter, for example. The support structure is configured so that it can expand when desired, such as by the expansion of the balloon catheter. The delivery systems used for such a stent should be provided with degrees of rotational and axial orientation capabilities to properly position the new stent at its desired location.

The wires of the support structure of the stents in other embodiments can alternatively be formed from a shape memory material such as a nickel titanium alloy (e.g., Nitinol). With this material, the support structure is self-expandable from a contracted state to an expanded state, such as by the application of heat, energy, or the like, or by the removal of external forces (e.g., compressive forces provided by a sheath). This support structure can typically be repeatedly compressed and re-expanded without damaging the structure of the stent. In some embodiments of the present disclosure, the stent 52 is made of a series of wires that are compressible and expandable through the application and removal of external forces, and may include a series of Nitinol wires that are approximately 0.011-0.015 inches in diameter, for example. The support structure of the stents may be laser cut from a single piece of material or may be assembled from a number of different components. For these types of stent structures, one example of a system that can be used for delivery thereof includes a catheter with a retractable sheath that covers the stent until it is to be deployed, at which point the sheath can be retracted to allow the stent to expand.

Valve structure 54 includes multiple leaflets 56 that are attached to stent features 58. The stent features 58 may be a separate component that is secured within the stent, or the stent features may actually be the general area where two leaflet pieces that are sewn to the stent form a "peak" or commissure area. The valve structures shown and described relative to the Figures are generally configured to accommodate multiple leaflets and replace a heart valve (e.g., heart valve 10) that has a corresponding number of commissure posts for a multiple-leaflet structure. The replacement prosthetic heart valves of the disclosure will generally include three leaflets, but can incorporate more or less than three leaflets.

As referred to herein, the replacement heart valves may include a wide variety of different configurations, such as a replacement heart valve having tissue leaflets or a synthetic heart valve having polymeric, metallic, or tissue-engineered leaflets, and can be specifically configured for replacing any heart valve.

The leaflets of the valves can be formed from a variety of materials, such as autologous tissue, xenograph material, or synthetics as are known in the art. The leaflets may be provided as a homogenous, biological valve structure, such as a porcine, bovine, or equine valve. Alternatively, the leaflets can be provided independent of one another (e.g., bovine or equine pericardial leaflets) and subsequently assembled to the support structure of the stent. In another alternative, the stent and leaflets can be fabricated at the same time, such as may be accomplished using high strength nano-manufactured NiTi films produced at Advanced Bio Prosthetic Surfaces (ABPS) of San Antonio, Texas, for example.

In more general terms, the combination of a support structure with one or more leaflets for a replacement heart valve can assume a variety of other configurations that differ from those shown and described, including any known prosthetic heart valve design. In some embodiments, the support structure with leaflets can be any known expandable prosthetic heart valve configuration, whether balloon expandable, self-expanding, or unfurling (as described, for example, in U.S. Patent Nos. 3,671,979; 4,056,854; 4,994,077; 5,332,402; 5,370,685; 5,397,351; 5,554,185; 5,855,601; and 6,168,614; U.S. Patent Application Publication No. 2004/0034411; Bonhoeffer P., et al., "Percutaneous Insertion of the Pulmonary Valve," Pediatric Cardiology, 2002; 39:1664-1669; Anderson H R, et al., 'Transluminal Implantation of Artificial Heart Valves," EUR Heart J., 1992; 13:704-708; Anderson, J. R., et al., "Transluminal Catheter Implantation of New Expandable Artificial Cardiac Valve," EUR Heart J., 1990, 11: (Suppl) 224a; Hilbert S. L., "Evaluation of Explanted Polyurethane Trileaflet Cardiac Valve Prosthesis," J Thorac Cardiovascular Surgery, 1989; 94:419-29; Block P C, "Clinical and Hemodyamic Follow-Up After Percutaneous Aortic Valvuloplasty in the Elderly," The American Journal of Cardiology, Vol. 62, Oct. 1, 1998; Boudjemline, Y., "Steps Toward Percutaneous Aortic Valve Replacement," Circulation, 2002; 105:775-558; Bonhoeffer, P., "Transcatheter Implantation of a Bovine Valve in Pulmonary Position, a Lamb Study," Circulation, 2000:102:813-816; Boudjemline, Y., "Percutaneous Implantation of a Valve in the Descending Aorta In Lambs," EUR Heart J, 2002; 23:1045-1049; Kulkinski, D., "Future Horizons in Surgical Aortic Valve Replacement: Lessons Learned During the Early Stages of Developing a Transluminal Implantation Technique," ASAIO J, 2004; 50:364-68).

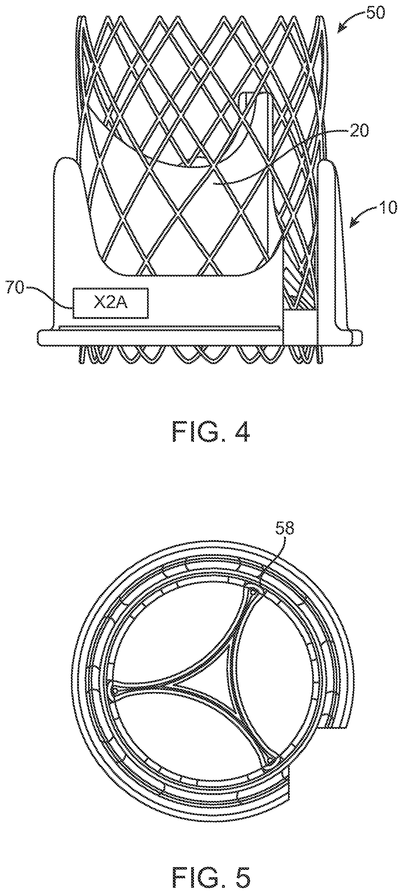

FIGS. 4-5 illustrate one embodiment of the replacement prosthetic heart valve 50 in combination with the original prosthetic heart valve 10, where the replacement prosthetic heart valve is positioned within the opening 20 of the original prosthetic heart valve. For illustration purposes, a portion of the stent structure 14 is removed so that that the opening of the heart valve 10 can be viewed more clearly, however, the stent structure 14 will typically be a continuous ring structure that has previously been implanted in a patient.

In some embodiments, the replacement valve 50 is delivered percutaneously to the area of the original heart valve 10. If the valve 50 includes a balloon-expandable stent, this can include providing a transcatheter assembly, including a delivery catheter, a balloon catheter, and a guide wire. Some delivery catheters of this type are known in the art, and define a lumen within which the balloon catheter is received. The balloon catheter, in turn, defines a lumen within which the guide wire is slideably disposed.

Further, the balloon catheter includes a balloon that is connected to an inflation source. It is noted that if the stent being implanted is a self-expanding type of stent, the balloon would not be needed and a sheath or other restraining means would be used for maintaining the stent in its compressed state until deployment of the stent, as described herein. In any case, for a balloon-expandable stent, the transcatheter assembly is appropriately sized for a desired percutaneous approach to the implantation location. For example, the transcatheter assembly can be sized for delivery to the heart valve via an opening at a carotid artery, a jugular vein, a sub-clavian vein, femoral artery or vein, or the like. Essentially, any percutaneous intercostals penetration can be made to facilitate use of the transcatheter assembly.

As mentioned herein, the information markers can be included with any suitable original heart prosthesis. For example, FIG. 6 is a schematic perspective view of an original heart prosthesis that includes an annuloplasty prosthesis 80. In the example illustrated in FIG. 6, the annuloplasty prosthesis 80 is a ring that defines an opening 84. Although prosthesis 80 takes the form of a ring, the prosthesis could take any suitable shape, e.g., a band. The annuloplasty prosthesis 80 also includes at least one information marker 82 positioned in any suitable location on or in the prosthesis. All of the design considerations and possibilities regarding the at least one information marker 70 of FIG. 1 applied equally to the at least one information marker 82 of FIG. 6.

The annuloplasty prosthesis 80 can include any suitable annuloplasty prosthesis. Further, the prosthesis 80 can be used to repair any suitable valve, e.g., aortic, mitral, pulmonic, venous, gastric, tricuspid, etc. And any suitable technique or combination of techniques can be used to implant the prosthesis 80 in a suitable location within a patient.

In some circumstances, a patient's valve that has been previously repaired using an annuloplasty prosthesis may require complete replacement with a replacement prosthetic heart valve, e.g. replacement prosthetic heart valve 50 of FIG. 2. In such circumstances, a less invasive approach can include leaving the annuloplasty prosthesis in place and positioning a replacement prosthetic heart valve in an opening defined by the annuloplasty prosthesis.

For example, FIG. 7 is a schematic side view of the replacement prosthetic heart valve 50 of FIG. 2 positioned relative to the annuloplasty prosthesis 80 of FIG. 6. As shown in FIG. 7, the replacement prosthetic heart valve 50 is positioned in the opening 84 defined by the annuloplasty prosthesis 80. Any suitable technique or combinations of techniques can be used to position the replacement prosthetic heart valve 50 in the opening 84 of the annuloplasty prosthesis 80.

In some embodiments, prior to delivery of the replacement prosthetic heart valve 50, a physician or clinician can detect the at least one information marker 70 of the original heart prosthesis (e.g., original prosthetic heart valve 10 of FIG. 1 or annuloplasty prosthesis 80 of FIG. 6). The replacement prosthetic heart valve 50 can be selected based on the information provided by the at least one information marker of the original heart prosthesis (e.g., at least one information marker 70 of original prosthetic heart valve 10 of FIG. 1 or at least one information marker 82 of annuloplasty prosthesis 80 of FIG. 6). For example, the at least one information marker 70 can indicate information regarding one or more of the manufacturer, type, model, feature, size, and date associated with the original heart prosthesis. The physician or clinician can use this information to determine an appropriate replacement prosthetic heart valve 50 that is compatible with the original heart prosthesis. Such information can be used to locate an appropriate replacement valve in a lookup table, software, etc., or other type of literature that provides guidance on the appropriate size, shape, model, etc. of replacement heart valve that has been qualified for re-intervention with the original heart valve.

Any suitable technique can be used to detect the at least one information marker of the original heart prosthesis. For example, in some embodiments, the at least one information marker can include radiopaque material such that the marker is detectable or readable using fluoroscopic visualization techniques.

Prior to delivery, the replacement stent is mounted over the balloon in a contracted state to be as small as possible without causing permanent deformation of the stent structure. As compared to the expanded state, the support structure is compressed onto itself and the balloon, thus defining a decreased inner diameter as compared to its inner diameter in the expanded state. While this description is related to the delivery of a balloon-expandable stent, the same basic procedures can also be applicable to a self-expanding stent, where the delivery system would not include a balloon, but would, in some embodiments, include a sheath or some other type of configuration for maintaining the stent in a compressed condition until its deployment.

With the stent mounted to the balloon, the transcatheter assembly is delivered through a percutaneous opening (not shown) in the patient via the delivery catheter. The implantation location is located by inserting the guide wire into the patient, which guide wire extends from a distal end of the delivery catheter, with the balloon catheter otherwise retracted within the delivery catheter. The balloon catheter is then advanced distally from the delivery catheter along the guide wire, with the balloon and stent positioned relative to the implantation location. In an alternative embodiment, the stent is delivered to an implantation location via a minimally invasive surgical incision (i.e., non-percutaneously). In another alternative embodiment, the stent is delivered via open heart/chest surgery.

While one exemplary embodiment of a replacement valve is described herein, it is understood that the stent of the replacement valve can have a structure that is at least somewhat different than that illustrated in FIG. 2. That is, the stent can have the same or a different number of crowns at its opposite ends, and/or the center portion can have a more or less dense concentration of wires than either of the ends. The stent may further include a central bulbous region between the first and second ends that has a larger diameter than the first and second ends of the stent. The bulbous region can be configured to generally match the contours of the anatomy where the stent will be positioned in the patient (e.g., at the aortic valve sinus region). The stent may alternatively or additionally include flared portions that extend from opposite sides of the central portion. Such a stent may be positioned within the anatomy (e.g., the aorta) of a patient so that the flares extend into the adjacent ventricle in order to help anchor the stent in place but so that they do not disrupt the native anatomical function.

It can be advantageous for the stent delivery process that the replacement valve is retractable or partially retractable back into a sheath at any point in the process until the stent is disengaged from the delivery system. This can be useful for repositioning of the stent if it is determined that the stent has been improperly positioned relative to the patient's anatomy and/or the original heart prosthesis into which it is being delivered.

As previously described, the at least one information marker can be made of any suitable material, e.g., radiopaque or radiopaque impregnated material. The radiopaque material selected for this purpose may be biocompatible. Such materials include tungsten, tantalum, platinum, gold, barium silicate, as well as alloys such as Hastelloy® metals.

Various processes exist for forming the radiopaque markers from such materials. In some embodiments, an etching process can be used to create the articles of the markers. This process may be a photo etching process whereby a photo-resistive coating is applied as a mask to a light-sensitive polymer plate. Light is projected onto the plate and the plates are then washed to remove the photo-resistive material that was used as the mask. An additional washing step may then be used to chemically remove the portion of the metal that was exposed to the light. In other embodiments, the photo-resistive coating and the exposed metal can be removed in one washing step. Other similar etching processes may be used as are known to those skilled in the art.

Another mechanism for creating the radiopaque articles for use in the described markers involves punching the articles from a sheet of radiopaque material. For instance, a ribbon of material may be fed into a die set having male and female portions that stamp out the characters. In one case, the punched articles may not be entirely separated one from another during the punching process but may remain connected to a larger sheet of such articles via break-away tabs. Prior to use, a desired article may be separated from the larger sheet of articles by twisting, bending, cutting, or otherwise breaking the respective tab. This allows the articles, which may individually be very small, to be readily stored and managed as a group until just prior to use. Such a punching process, as well as the use of break-away tabs, may produce radiopaque articles having jagged edges and/or burrs.

Yet another technique for producing the radiopaque articles involves using a laser cutting technique. Laser cutting can produce very tight tolerances and smooth edges, aiding readability of small radiopaque markers. Some materials, however, may be expensive or difficult to process using this technique. In particular, this technique may be expensive at higher volume production levels.

Still another option for creating the radiopaque articles involves a sintering process. According to this technique, powdered radiopaque material mixed with glue is pressed into a form and baked until all of the glue has been dissipated and the radiopaque particles bind together. This type of process creates a porous structure which may more readily adhere to the molecules of a polymer used during a subsequent molding process, with the degree to which the polymer is received by the pores being dependent upon molecular size of the polymer.

Metal injection molding can also be used to create the radiopaque articles. In this scenario, a radiopaque powder or slurry is injected under pressure into a mold. The powder or slurry is then baked until the radiopaque particles bind one to another. As with sintering, this may produce a relatively more porous radiopaque article.

Further, in some embodiments, radiopaque impregnated sutures can be used to make suture lines or patterns on a valve to create a type of marker associated with the valve.

Illustrative embodiments of this disclosure are discussed and reference has been made to possible variations within the scope of this disclosure. These and other variations and modifications in the disclosure will be apparent to those skilled in the art without departing from the scope of the disclosure, and it should be understood that this disclosure is not limited to the illustrative embodiments set forth herein. Accordingly, the disclosure is to be limited only by the claims provided below.

## Claims

1. A heart prosthesis comprising at least one information marker (70, 82) that indicates one or more of a manufacturer, type, model, size, and date associated with the heart prosthesis, wherein the at least one information marker is visible with fluoroscopic visualization techniques, wherein the heart prosthesis comprises a prosthetic heart valve (10),
wherein the information marker is positioned on a sealing skirt of the prosthetic heart valve, and
wherein the at least one information marker comprises a QR code.

2. The heart prosthesis of claim 1, wherein the heart prosthesis comprises a surgically implanted prosthetic heart valve (10).

3. The heart prosthesis of claim 1, wherein the at least one information marker comprises multiple markers.

4. A replacement prosthetic heart valve (50) in combination with an original heart prosthesis, wherein the original heart prosthesis is a heart prosthesis according to claim 1.

5. The combination of claim 4, wherein the at least one information marker comprises multiple markers.

## Patentansprüche

1. Herzprothese, umfassend mindestens einen Informationsmarker (70, 82), der eines oder mehrere aus einem Hersteller, Typ, Modell, Größe und Datum, die mit der Herzprothese assoziiert sind, anzeigt, wobei der mindestens eine Informationsmarker mit fluoroskopischen Visualisierungstechniken sichtbar ist, wobei die Herzprothese eine prothetische Herzklappe (10) umfasst,
wobei der Informationsmarker auf einem Dichtungsmantel der prothetischen Herzklappe positioniert ist, und
wobei der mindestens eine Informationsmarker einen QR-Code umfasst.

2. Herzprothese nach Anspruch 1, wobei die Herzprothese eine chirurgisch implantierte prothetische Herzklappe umfasst (10).

3. Herzprothese nach Anspruch 1, wobei der mindestens eine Informationsmarker mehrere Marker umfasst.

4. Ersatz-Herzklappenprothese (50) in Kombination mit einer Originalprothese, wobei die Original-Herzprothese eine Herzprothese nach Anspruch 1 ist.

5. Kombination nach Anspruch 4, wobei der mindestens eine Informationsmarker mehrere Marker umfasst.

## Revendications

1. Prothèse cardiaque comprenant au moins un marqueur d'information (70, 82) qui indique un ou plusieurs parmi le fabricant, le type, le modèle, la taille et la date associés à la prothèse cardiaque, dans laquelle le au moins un marqueur d'information est visible avec des techniques de visualisation fluoroscopique, dans laquelle la prothèse cardiaque comprend une valvule cardiaque prothétique (10),
dans laquelle le marqueur d'information est positionné sur une jupe d'étanchéité de la valvule cardiaque prothétique, et
dans laquelle le au moins un marqueur d'information comprend un QR code.

2. Prothèse cardiaque selon la revendication 1, dans laquelle la prothèse cardiaque comprend une valvule cardiaque prothétique (10) implantée par voie chirurgicale.

3. Prothèse cardiaque selon la revendication 1, dans laquelle le au moins un marqueur d'information comprend plusieurs marqueurs.

4. Valvule cardiaque prothétique (50) de remplacement en combinaison avec une prothèse cardiaque d'origine, dans laquelle la prothèse cardiaque d'origine est une prothèse cardiaque selon la revendication 1.

5. Combinaison selon la revendication 4, dans laquelle le au moins un marqueur d'information comprend plusieurs marqueurs.
